# EUROPEAN PATENT SPECIFICATION

(11) **EP 0 533 243 B1**
(45) Date of publication and mention of the grant of the patent: **17.12.1997**
(21) Application number: 92202723.0
(22) Date of filing: 08.09.1992
(51) Int. Cl.: C07D 401/12, C07D 471/10, A61K 31/445, C07D 401/14

(54) **Hydantoin and succinimide-substituted spiroindanylcamphorsulfonyl derivatives**
Hydantoin und Succinimid-substituierte Spiroindanylcamphorsulfonyl als Oxytocin-Antagonisten
Dérivés de spiroindanylcamphorsulfonyl substitués par un radical hydantoine ou succinimide comme antagonists d'oxytocine

(30) Priority: 16.09.1991 US 760416
(43) Date of publication of application: 24.03.1993
(73) Proprietor: Merck & Co., Inc., Rahway New Jersey 07065-0900 (US)
(72) Inventor: Gilbert, Kevin, Bally, PA 19503 (US); Williams, Peter D., Harleysville, PA 19438 (US); Hobbs, Doug W., Lansdale, PA 19446 (US); Evans, Ben E., Lansdale, PA 19446 (US); Veber, Daniel F., Ambler, PA 19002 (US)
(74) Representative: Thompson, John Dr.

(56) References cited:
- EP-A- 0 444 945
- EP-A- 0 445 974
- EP-A- 0 450 761
- EP-A- 0 486 280
- US-A- 3 654 287
- EP-A- 0 444 945
- EP-A- 0 445 974
- EP-A- 0 450 761
- EP-A- 0 486 280
- US-A- 3 654 287

## Description

### FIELD OF THE INVENTION

The present invention provides novel compounds, novel compositions, methods of their use and methods of their manufacture, such compounds generally pharmacologically useful as agents in obstetric and gynecologic therapy. The aforementioned pharmacologic activities are useful in the treatment of mammals. More specifically, the compounds of the present invention can be used in the treatment of preterm labor, stopping labor preparatory to Cesarean delivery, and in the treatment of dysmenorrhea. At the present time, there is a need in the area of obstetric and gynecologic therapy for such agents.

### BACKGROUND OF THE INVENTION

In the field of obstetrics, one of the most important problems is the management of preterm labor. A significant number of the pregnancies progressing past 20 weeks of gestation experience premature labor and delivery, which is a leading cause of neonatal morbidity and mortality. Despite major advances in neonatal care, retention of the fetus in utero is preferred in most instances.

Tocolytic (uterine-relaxing) agents that are currently in use include β₂-adrenergic agonists, magnesium sulfate and ethanol. Ritodrine, the leading β₂-adrenergic agonist, causes a number of cardiovascular and metabolic side effects in the mother, including tachycardia, increased renin secretion, hyperglycemia (and reactive hypoglycemia in the infant). Other β₂-adrenergic agonists, including terbutaline and albuterol have side effects similar to those of ritodrine. Magnesium sulfate at plasma concentrations above the therapeutic range of 4 to 8 mg/dL can cause inhibition of cardiac conduction and neuromuscular transmission, respiratory depression and cardiac arrest, thus making this agent unsuitable when renal function is impaired. Ethanol is as effective as ritodrine in preventing premature labor, but it does not produce a corresponding reduction in the incidence of fetal respiratory distress that administration of ritodrine does.

It has been proposed that a selective oxytocin antagonist would be the ideal tocolytic agent. In the last few years, evidence has accumulated to strongly suggest that the hormone oxytocin is the physiological initiator of labor in several mammalian species including humans. Oxytocin is believed to exert this effect in part by directly contracting the uterine myometrium and in part by enhancing the synthesis and release of contractile prostaglandins from the uterine endometrium/decidua. These prostaglandins may, in addition, be important in the cervical ripening process. By these mechanisms, the process of labor (term and preterm) is initiated by a heightened sensitivity of the uterus to oxytocin, resulting in part as a result of a well-documented increase in the number of oxytocin receptors in this tissue. This "up-regulation" of oxytocin receptors and enhanced uterine sensitivity appears to be due to trophic effects of rising plasma levels of estrogen towards term. By blocking oxytocin, one would block both the direct (contractile) and indirect (enhanced prostaglandin synthesis) effects of oxytocin on the uterus. A selective oxytocin blocker, or antagonist, would likely be more efficacious for treating preterm labor than current regimens. In addition, since oxytocin at term has major effects only on the uterus, such a oxytocin antagonizing compound would be expected to have few, if any, side effects.

The compounds of the present invention can also be useful in the treatment of dysmenorrhea. This condition is characterized by cyclic pain associated with menses during ovulatory cycles. The pain is thought to result from uterine contractions and ischemia, probably mediated by the effect of prostaglandins produced in the secretory endometrium. By blocking both the direct and indirect effects of oxytocin on the uterus, a selective oxytocin antagonist can be more efficacious for treating dysmenorrhea then current regimens.

It is, therefore, a purpose of this invention to provide substances which more effectively antagonize the function of oxytocin in disease states in animals, preferably mammals, especially in humans. It is another purpose of this invention to prepare novel compounds which more selectively inhibit oxytocin. It is still another purpose of this invention to provide a method of antagonizing the functions of oxytocin in disease states in mammals. It is also a purpose of this invention to develop a method of preventing or treating oxytocin-related disorders of preterm labor and dysmenorrhea by antagonizing oxytocin.

It has now been found that compounds of formula I are antagonists of oxytocin and bind to the oxytocin receptor. When the oxytocin receptor is bound by the compounds of the present invention, oxytocin is antagonized by being blocked from its receptor and thus being unable to exert its biologic or pharmacologic effects. These compounds are useful in the treatment and prevention of oxytocin-related disorders of animals, preferably mammals and especially humans. These disorders are primarily preterm labor and dysmenorrhea. The compounds would also find usefulness for stoppage of labor preparation to Cesarean delivery.

The compounds of the present invention are those of the general structural formula: 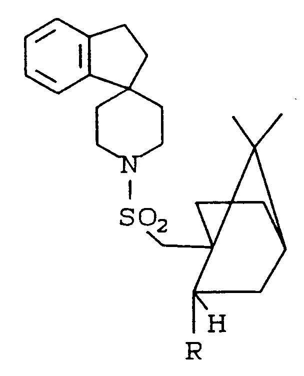 and the pharmaceutically acceptable salts thereof,
wherein
R is
Het-R', wherein
Het is
substituted saturated or unsaturated 5 or 6 membered heterocyclic rings containing 1, 2 or 3 heteroatoms, wherein said heteroatoms are N, O or S;
R' is independently one or more of hydrogen, oxo, thiono, amino or unsubstituted or substituted alkyl, where said alkyl substituent is independently one or more of hydroxyl, amino, oxo, sulfonyl, alkylsulfonyl, carboxyl, phenyl, indole, alkoxycarbonyl, carbonylamino, guanidino, alkoxycarbonylamino, imidazole, imidazolylalkylaminocarbonyl, pyrrolidine,tetrazolylaminocarbonyl, tetrazolylalkylcarbonylamino, alkylaminoalkylaminocarbonyl and dialkylaminoalkylaminocarbonyl.

Salts encompassed within the term "pharmaceutically acceptable salts" refer to non-toxic salts of the compounds of this invention which are generally prepared by reacting the free base with a suitable organic or inorganic acid. Representative salts include the following salts:

| | |
|---|---|
| Acetate | Lactobionate |
| Benzenesulfonate | Laurate |
| Benzoate | Malate |
| Bicarbonate | Maleate |
| Bisulfate | Mandelate |
| Bitartrate | Mesylate |
| Borate | Methylbromide |
| Bromide | Methylnitrate |
| Calcium Edetate | Methylsulfate |
| Camsylate | Mucate |
| Carbonate | Napsylate |
| Chloride | Nitrate |
| Clavulanate | N-methylglucamine |
| Citrate | Oxalate |
| Dihydrochloride | Pamoate (Embonate) |
| Edetate | Palmitate |
| Edisylate | Pantothenate |
| Estolate | Phosphate/diphosphate |
| Esylate | Polygalacturonate |
| Fumarate | Salicylate |
| Gluceptate | Stearate |
| Gluconate | Subacetate |
| Glutamate | Succinate |
| Glycollylarsanilate | Tannate |
| Hexylresorcinate | Tartrate |
| Hydrabamine | Teoclate |
| Hydrobromide | Tosylate |
| Hydrocloride | Triethiodide |
| Hydroxynaphthoate | Valerate |
| Iodide | |
| Isethionate | |
| Lactate | |

The term "pharmacologically effective amount" shall mean that amount of a drug or pharmaceutical agent that will elicit the biological or medical response of a tissue, system, animal or human that is being sought by a researcher or clinician.

The term "alkyl" shall mean straight or branched chain alkanes, alkenes and alkynes with one or more degrees of unsaturation at any position on the chain, of one to ten total carbon atoms or any number within this range.

The term "aryl" shall mean phenyl.

The term "cycloalkyl" shall mean cyclic rings of alkanes, alkenes or alkynes with one or more degrees of unsaturation at any position of the ring, of three to eight total carbon atoms.

Whenever the terms "alkyl" or "aryl" or either of their prefix roots appear in a name of a substituent (e.g. aralkoxyaryloxy) they shall be interpreted as including those limitations given above for "alkyl" and "aryl". Designated numbers of carbon atoms (e.g. C₁₋₁₀) shall refer independently to the number of carbon atoms in an alkyl or cyclic alkyl moiety or to the alkyl portion of a larger substituent in which alkyl appears as its prefix root.

The term "oxo" shall refer to the substituent =O.

The term "halogen" shall include iodine, bromine, chlorine and fluorine.

The term "preterm labor" shall mean expulsion from the uterus of a viable infant before the normal end of gestation, or more particularly, onset of labor with effacement and dilation of the cervix before the 37th week of gestation. It may or may not be associated with vaginal bleeding or rupture of the membranes.

The term "dysmenorrhea" shall mean painful menstruation.

The term "cesarean delivery" shall mean incision through the abdominal and uterine walls for delivery of a fetus.

The term "substituted" shall be deemed to include multiple degrees of substitution by a named substitutent.

The ability of the compounds of formula I to antagonize oxytocin makes these compounds useful as pharmacologic agents for mammals, especially for humans, for the treatment and prevention of disorders wherein oxytocin may be involved. Examples of such disorders include preterm labor and especially dysmenorrhea. These compounds may also find usefulness for stoppage of labor preparatory to Cesarean delivery.

Because of the known relationship of vasopressin to oxytocin, the compounds of the present invention are also useful as vasopressin antagonists. Vasopressin antagonists are useful in the treatment or prevention of disease states involving vasopressin disorders, including their use as diuretics and their use in congestive heart failure.

The compounds of the present invention can be administered in such oral dosage forms as tablets, capsules (each including timed release and sustained release formulations), pills, powders, granules, elixers, tinctures, suspensions, syrups and emulsions. Likewise, they may also be administered in intravenous (both bolus and infusion), intraperitoneal, subcutaneous or intramuscular form, all using forms well known to those of ordinary skill in the pharmaceutical arts. An effective but non-toxic amount of the compound desired can be employed as a tocolytic agent.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Oral dosages of the present invention, when used for the indicated effects, will range between about 0.3-6.0 gm/day orally. Intravenously, the most preferred doses will range from 0.1 to about 10 mg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittant throughout the dosage regimen.

In the methods of the present invention, the compounds herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, zanthan gum and the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

The compounds of formula I can be prepared readily according to the following reaction Schemes (in which all variables are as defined before) and Examples or modifications thereof using readily available starting materials, reagents and conventional synthesis procedures. In these reactions, it is also possible to make use of variants which are themselves known to those of ordinary skill in this art, but are not mentioned in greater detail.

The most preferred compounds of the invention are any or all of those specifically set forth in these examples. These compounds are not, however, to be construed as forming the only genus that is considered as the invention, and any combination of the compounds or their moieties may itself form a genus. The following examples further illustrate details for the preparation of the compounds of the present invention. Those skilled in the art will readily understand that known variations of the conditions and processes of the following preparative procedures can be used to prepare these compounds. All temperatures are degrees Celsius unless noted otherwise.

Abbreviations used in the Examples are as follows:
- TEA: = triethylamine
- DIEA: = diisopropylethylamino
- BOP: = benzotriazol-yloxytris(dimethylamino) phosphonium hexafluorophosphate
- THF: = tetrahydrofuran
- DMF: = dimethylformamide
- LAH: = lithium aluminum hydride
- TFA: = trifluoroacetic acid
- HPLC Method A: = 15 min. linear gradient
95:5 A:B to 0:100 A:B
A - H₂O containing 0.1% by vol. TFA
B = CH₃CN containing 0.1% by vol. TFA
2.0 mL/min flow rate
12 cm C₁₈ reverse phase column
UV detection (215 nm)

TLC was performed on 20 cm plates coated with silica gel (250 microns) from Analtech.

### EXAMPLE A

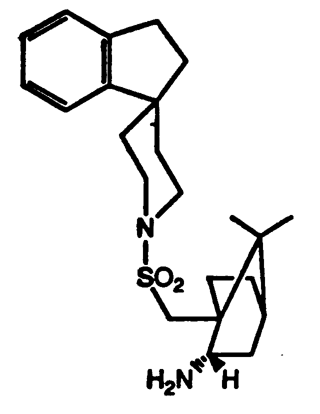

### Endo-(1S)-1'(((2-amino-7,7-dimethylbicyclo(2.2.1)-hept-1-yl)-methyl)-sulfonyl)spiro(1H-indan-1,4'-piperidine)

Di-t-butyl dicarbonate (31g, 0.14 mole available from Aldrich) and bis(2-chloroethyl) amine hydrochloride (21.6g, 0.12 mole Aldrich) were combined in CH₂Cl₂ (250 ml) stirred at ambient temperature and treated with triethylamine (12.8 g, 0.127 mole) added dropwise over 15 minutes. After 1 hour, another 1.5 ml of triethylamine was added. After a total of 2.5 hours, the mixture was poured onto a silica gel column packed with CH₂Cl₂:hexane (1:1), and eluted with CH₂Cl₂. The combined product fractions were evaporated to dryness in vacuo to give N,N-bis(2-chloroethyl)-t-butyl-carbamate.

To a solution of indene (10.3 g, 89 mmole) in dry tetrahydrofuran (THF, 18 ml) cooled in an ice bath and maintained under a nitrogen blanket was added lithium bis(trimethylsilyl)amide (Aldrich, 177 ml of a 1.0M solution in THF; 177 mmole) over 15 minutes. The mixture was stirred in the cold for 30 minutes, then added over 15 minutes to a solution of N,N-bis(2-chloroethyl)-t-butylcarbamate (21.2 g, 88 mmole) stirred in an ice bath. The mixture was stirred for 2 hours in the cold and for 30 minutes at ambient temperature under nitrogen, then evaporated in vacuo to a foam. CH₂Cl₂ was added and the resulting mixture poured onto a silica gel column packed with 40% hexane in CH₂Cl₂. The column was eluted with 40% hexane in CH₂Cl₂ followed by CH₂Cl₂, and the product fractions were evaporated to dryness in vacuo to provide 1'-(t-butyloxycarbonyl) -spiro(indene-1,4'-piperidine).

1'-(t-Butyloxycarbonyl)spiro(indene-1,4'-piperidine) (16 g, 56 mmole) in ethyl acetate (250 ml) was stirred in an ice bath and saturated with HCl(g) for 30 minutes. The mixture was evaporated to dryness. Ethyl acetate was added and removed in vacuo three times, and the residue was triturated with diethyl ether and filtered to provide spiro(1H-indene-1,4'-piperidine) hydrochloride. The free base was obtained by slurrying the hydrochloride in aqueous sodium bicarbonate solution and extracting with CH₂Cl₂. The organic layer was separated, dried over sodium sulfate, filtered, and evaporated to dryness in vacuo to provide spiro(1H-indene-1,4'piperidine.

Spiro(1H-indene-1,4'piperidine) (308 mg, 1.66 mmol) and (+)-10-camphorsulfonyl chloride (418 mg, 1.66 mmol) were combined in CH₂Cl₂ and treated with triethylamine (0.23 ml). The mixture was stirred at ambient temperature for 15 minutes, then poured onto a silica gel column and eluted with 1:1 CH₂Cl₂:hexane. The product fractions were combined and evaporated to dryness in vacuo to provide (1S)-1'-(((7,7-dimethyl-2-oxobicicylo-(2.2.1) hept-1-yl)-methyl)sulfonyl)spiro(1H-indene-1.4'-piperidine) as a solid which was recrystallized from petroleum ether and dried overnight in vacuo at ambient temperature.

(1S)-1'-(((7,7-dimethyl-2-oxobicyclo(2.2.1) hept-1-yl)methyl)sulfonyl)spiro(1H-indene-1,4'-piperidine) (30 g, 0.075 mole) in pyridine (500 mL) was heated in an oil bath to 70°C (internal). Hydroxylamine hydrochloride (30 g) was added in three portions over ca. 20 minutes. After 2 hours, an additional 10 g of hydroxylamine hydrochloride was added (over 10 minutes). At 30, 40, and 50 minutes additional elapsed time, further 3 g lots of hydroxylamine hydrochloride were added. After another 30 minutes, the mixture was poured into water (2 L) and extracted 3 times with ethyl acetate (300 mL portions). The organic layers were combined, washed with 1N HCl (600 mL total), dried over sodium sulfate, filtered, and evaporated to dryness in vacuo. EtOH (abs; ca. 250 mL) was added to the resulting thick syrup and the solution allowed to stand at ambient temperature overnight. The mixture was filtered and the filtrate boiled down to ca. 80 mL. After standing, the mixture was again filtered and boiled down to ca. 20 mL. After a third filtration, the filtered solids were combined to give (1S)-1'-(((7, 7-dimethyl-2-oximinobicyclo(2.2.1)hept-1-yl)-methyl) sulfonyl)spiro(1H-indene-1,4'-piperidine) (28 g).

Freshly prepared, activated Raney Nickel catalyst (ca. 30 g) in water was allowed to settle and the water decanted. Abs. ethanol (300 mL) was added, and the mixture swirled and again allowed to settle. The solvent was decanted. Two more wash-decant cycles with 150 mL of ethanol were similarly carried out. (1S)-1'-(((7,7-dimethyl-2-oximinobicyclo (2.2.1)hept-1-yl)methyl)sulfonyl)-spiro(1H-indene-1, 4'-piperidine) (30 g) was stirred in a mixture of abs. ethanol (450 mL) and 2-methoxyethanol (900 mL), nitrogen was bubbled through the suspension/solution, and the Raney Nickel catalyst was added. The mixture was hydrogenated under 50 psi overnight. TLC (9:1 CH₂Cl₂MeOH, silica gel) showed the reaction to be complete. The catalyst was removed by filtration, and the filtrate evaporated to dryness in vacuo. The crude solid (27 g) was divided into 7 g batches, and each batch was dissolved in methylene chloride (ca. 200 mL) and flash chromatographed on silica (700 g in a 100 mm column, packed and eluted with 8% (v/v) methanol in methylene chloride), taking 200 mL fractions. The exo isomer of the title amine was obtained in fractions ca. 5-7, and the desired endo isomer in fractions ca. 8-16. TLC was on silica, eluted with 8% methanol-methylene chloride, phosphomolybdic acid stain. The combined product fractions were evaporated to dryness to provide the title compound (4.5 g from each 7 g lot, ca. 18 g total) as a colorless solid.

### EXAMPLE 1

### (1S)-1'-(((7,7-dimethyl-2-endo-(4-nitrophenyloxycarbonylamino)-bicyclo-(2.2.1)-hept-1-yl)-methyl)-sulfonyl)spiro(1H-indene-1,4'-piperdine)

The product of Example A [3.47 mmol] and 4-nitrophenyl chloroformate [3.64 mmol] were combined in THF. The reaction mixture was treated with triethylamine [4.54 mmol] and allowed to stir for 2 hours. The reaction mixture was concentrated to dryness and the resulting residue was purified by a silica gel column, while eluting with 1% ethyl acetate in methylene chloride. The product fractions were combined and concentrated to dryness in vacuo. The title compound was obtained as a white solid from ether.

### EXAMPLE 2

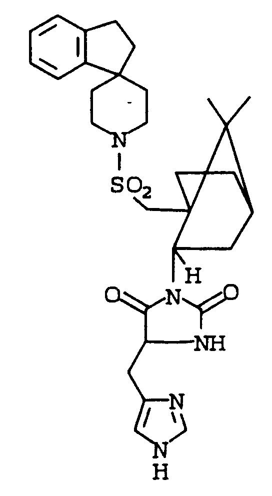

(1S)-1'-(((7,7-dimethyl-2-endo-(4-nitrophenyloxycarbonylamino)-bicyclo-(2.2.1)-hept-1-yl)-methyl)) sulfonyl)spiro(1H-indene-1,4'-piperdine) [1.80 mmol] and histidine methyl ester dihydrochloride [1.90 mmol] were combined in DMF. The reaction mixture was treated with triethylamine [5.90 mmol] and allowed to stir for 2 hours. The reaction mixture was concentrated to dryness and the resulting residue was dissolved in CH₂Cl₂. This CH₂Cl₂ solution was placed on a silica gel column and eluted with 2% methanol in CH₂Cl₂ and then with 95/5/0.5 of CH₂Cl₂/methanol/ammonium hydroxide. The product fractions were combined and evaporated to dryness in vacuo. A white solid was obtained from ether. The resulting white solid [0.954 mmol] and sodium hydride [0.45 mmol] were combined in ethanol and left stir 12 hours. The reaction mixture was concentrated to dryness and the resulting residue was dissolved in CH₂Cl₂. This solution was placed on a silica gel column and eluted with 95/5/0.5 of CH₂Cl₂/methanol/ammonium hydroxide. The product fractions were combined and evaporated to dryness in vacuo. The title compound was obtained as a white solid from ether and dried in vacuo, overnight.
m.p.: 146°C-192°C
NMR: Consistent with structure
HPLC: >99% pure
MS: M+H⁺=566.2 (FAB)
CHN:

| Calc'd for C₃₀H₃₉N₅O₄S·0.05 C₄H₁₀O·0.80 H₂O; | | | |
|---|---|---|---|
| | C, 62.12; | H, 7.10; | N, 12.00. |
| Found: | C, 62.10; | H, 7.02; | N, 12.01. |

### EXAMPLE 3

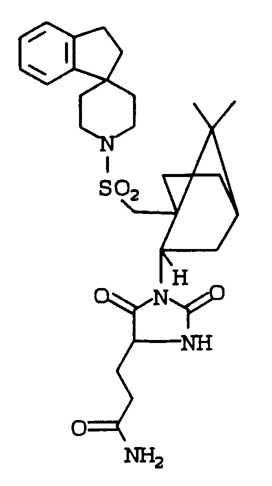

The procedure of Example 2 was carried out using the product of Example 1 [0.197 mmol] triethylamine [0.54 mmol] and substituting glutamine-t-butyl ester hydrochloride [0.217 mmol] for histidinne methyl ester dihydrochloride. Chromatographic elution for column 1 was with 1% methanol in CH₂Cl₂ and then with 3% methanol in CH₂Cl₂. The title compound was obtained as a white solid from ether and dried in vacuo, overnight.
mp: 104°-166°C
NMR: Consistent with structure
HPLE: >97% pure
MS: M+H⁺=557.2 (FAB)
CHN:

| Calc'd for C₂₉H₄₀N₄O₅S·0.50 C₄H₁₀O·0.10 H₂O; | | | |
|---|---|---|---|
| | C, 62.51; | H, 7.65; | N, 9.41. |
| Found: | C, 62.55; | H, 7.36; | N, 9.04. |

### EXAMPLE 4

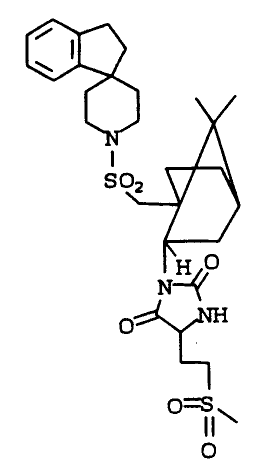

The procedure of Example 2 was carried out using the product of Example 1 [0.215 mmol], triethylamine [0.55 mmol] and substituting L-methionine methyl ester [0.239 mmol] for histidine methyl ester dihydrochloride. Chromatographyic elution for column 1 was with 96/4/0.4 of CH₂Cl₂/methanol/ammonium hydroxide. For column 2, the elution was done with 5% methanol in CH₂Cl₂ and then with 95/5/0.5 of CH₂Cl₂/methanol/ammonium hydroxide. A white solid was obtained from ether. This white solid was dissolved in methanol. This solution was treated with oxone [0.284 mmol], which had been dissolved in a small amount of water, and the mixture was stirred at ambient temperature for 4 hours. The reaction mixture was concentrated and the resulting residue was partitioned between ethyl acetate and sat'd sodium bicarbonate solution. The ethyl acetate layer was dried over sodium sulfate, filtered, and the filtrate was concentrated in vacuo. The residue was purified by a silica gel column, eluted with 2% methanol in CH₂Cl₂. The product fractions were combined and concentrated. The title compound was obtained as a white solid from ether, and dried in vacuo, overnight.
m.p.: 134°-209°C
NMR: Consistent with structure
HPLC: >97% pure
MS: M+H⁺=592 (FAB)
CHN:

| Calc'd for C₂₉H₄₁N₃O₆S₂; | | | |
|---|---|---|---|
| | C, 58.86; | H, 6.98; | N, 7.10. |
| Found: | C, 58.55; | H, 6.59; | N, 7.04. |

### EXAMPLE 5

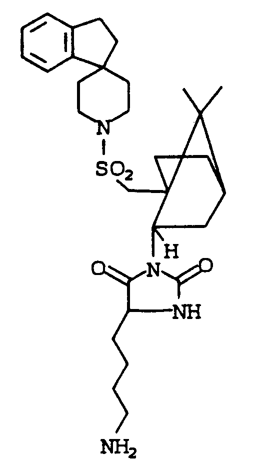

The procedure of Example 2 was carried out using the product of Example 1 [0.366 mmol], triethylamine [0.83 mmol], and substituting N-α-Cbz-L-Lysine methyl ester [0.379 mmol] for histidine methyl ester dihydrochloride. Chromatographic elution for column 1 was with 95/5/0.5 of CH₂Cl₂/methanol/ammonium hydroxide. For column 2, the elution was done with 2% methanol in CH₂Cl₂. A white solid was obtained from ether. This white solid was combined with palladium hydroxide on carbon catalyst in absoute ethanol. The mixture was hydrogenated at 40 p.s.i. overnight. The reaction mixture was filtered and the filtrate was concentrated to dryness. The resulting residue ws purified by a silica gel column, eluting with 92/8/0.8 of CH₂Cl₂/methanol/ammonium hydroxide. The product fractions were combined and evaporated to dryness. The title compound was obtained as a white solid from ether and was dried in vacuo, overnight. m.p.: 99°-158°C
NMR: Consistent with structure
HPLE: >94% pure
MS: M+H⁺=557.3 (FAB)
CHN:

| Calc'd for C₃₀H₄₄N₄O₄S·0.25 C₄H₁₀O·H₂O; | | | |
|---|---|---|---|
| | C, 64.11; | H, 8.18; | N, 9.65. |
| Found: | C, 64.12; | H, 8.01; | N, 9.32. |

### EXAMPLE 6

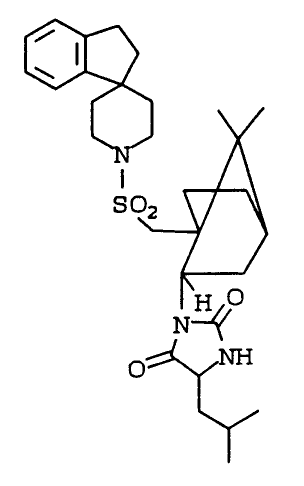

The procedure of Example 2 was carried out using the product of Example 1 [0.33 mmol], triethylamine [0.88 mmol], and substituting L-leucine methyl ester [0.35 mmol] for histidine methyl ester dihydrochloride. Chromatographic elution for column 1 was with 95/5/0.5 of CH₂Cl₂/methanol/ammonium hydroxide. For column 2, the elution was done with 1% methanol in CH₂Cl₂. The title compound was obtained as a white solid from ether and dried in vacuo, overnight.
m.p.: 106°-128°C
NMR: Consistent with structure
HPLE: >94% pure
MS: M+H⁺=542.3 (FAB)
CHN:

| Calc'd for C₃₀H₄₃N₃O₄S; | | | |
|---|---|---|---|
| | C, 66.51; | H, 8.00; | N, 7.76. |
| Found: | C, 66.24; | H, 8.10; | N, 7.49. |

### EXAMPLE 7

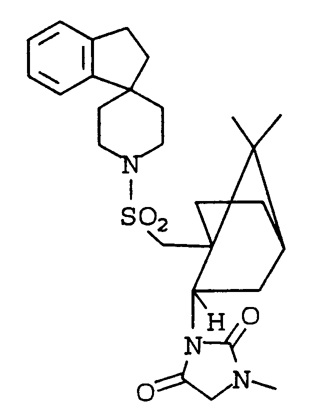

The procedure of Example 2 was carried out using the product of Example 1 [0.23 mmol], triethylamine [0.73 mmol], and substituting sarcosine ethyl ester [0.29 mmol] for histidine methyl ester dihydrochloride. Chromatographic elution for column 1 was with 1% ether in CH₂Cl₂ and then with 5% methanol in CH₂Cl₂. For column 2, elution was done with 25% ethyl acetate in hexane. The title compound was obtained as a white solid from ether and dried in vacuo, overnight.
m.p.: 89°-152°C
NMR: Consistent with structure
HPLE: >96% pure
MS: M+H⁺=500 (FAB)
CHN:

| Calc'd for C₂₇H₃₇N₃O₄S·0.10 C₄H₁₀O·0.40 H₂O; | | | |
|---|---|---|---|
| | C, 63.99; | H, 7.60; | N, 8.17. |
| Found: | C, 63.95; | H, 7.37; | N, 7.92. |

### EXAMPLE 8

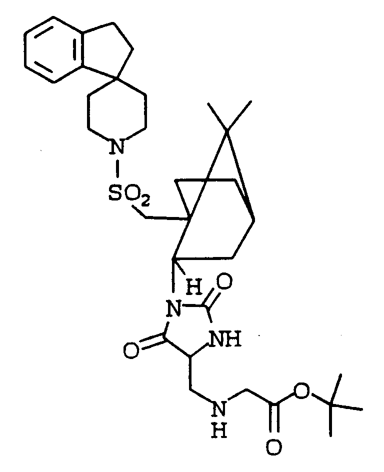

The procedure of Example 2 was carried out using the product of Example 1 [1.16 mmol], triethylamine [1.56 mmol], and substituting methyl(2-amino-3-(t-Boc-amino)) propanoate [1.27 mmol] for histidine methyl ester dihydrochloride. Chromatographic elution for column 1 was with 5% ether in CH₂Cl₂ and then with 3% methanol in CH₂Cl₂. For column 2, elution was done with 1% methanol in CH₂Cl₂. The title compound was obtained as a white solid from ether and dried in vacuo, overnight.
m.p.: 104°-176°C
NMR: Consistent with structure
HPLE: >97% pure
MS: M+H⁺=615 (FAB)
CHN:

| Calc'd for C₃₂H₄₆N₄O₆S·0.10 C₄H₁₀O·0.45 H₂O; | | | |
|---|---|---|---|
| | C, 61.73; | H, 7.66; | N, 8.89. |
| Found: | C, 61.68; | H, 7.66; | N, 8.97. |

### EXAMPLE 9

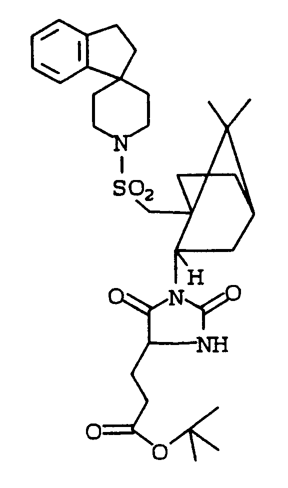

The procedure for Example 2 was carried out using the product of Example 1 [0.27 mmol], triethylamine [0.76 mmol], and substituting glutamic acid-α-methyl ester-α-methyl ester-α-t-butylester [0.308 mmol] for histidine methyl ester dihydrochloride. Chromatographic elution for column 1 was with 5% ether in CH₂Cl₂ and then with 5% methanol in CH₂Cl₂. For column 2, elution was done with 4% methanol in CH₂Cl₂. The title compound was obtained as a white solid from ether and dried in vacuo, overnight.
m.p.: 94°-117°C
NMR: Consistent with structure
HPLE: >93% pure
MS: M+H⁺=614 (FAB)
CHN:

| Calc'd for C₃₃H₄₇N₃O₆S·0.10 C₄H₁₀O·0.50 H₂O; | | | |
|---|---|---|---|
| | C, 63.65; | H, 7.84; | N, 6.67. |
| Found: | C, 63.68; | H, 7.64; | N, 6.67. |

### EXAMPLE 10

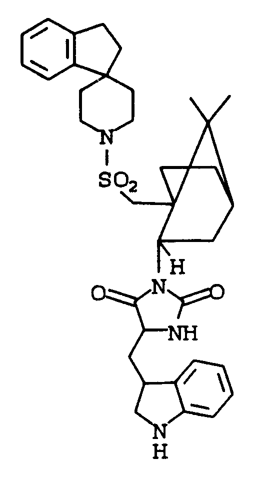

The procedure of Example 2 was carried out using the product of Example 1 [0.22 mmol], triethylamine [0.60 mmol], and substituting D-tryptophan methyl ester [0.24 mmol] for histidine methyl ester dihydrochloride. Chromatographic elution for column 1 was with 1% ether in CH₂Cl₂ and then with 5% methanol in CH₂Cl₂. For column 2, elution was done with 4% methanol in CH₂Cl₂. The title compound was obtained as a white solid from ether and dried in vacuo, overnight.
m.p.: 111°-176°C
NMR: Consistent with structure
HPLE: >92% pure
MS: M+H⁺=615.2 (FAB)
CHN:

| Calc'd for C₃₅H₄₂N₄O₄S·0.50 C₄H₁₀O·0.85 H₂O; | | | |
|---|---|---|---|
| | C, 66.62; | H, 7.29; | N, 8.49. |
| Found: | C, 66.64; | H, 6.93; | N, 8.12. |

### EXAMPLE 11

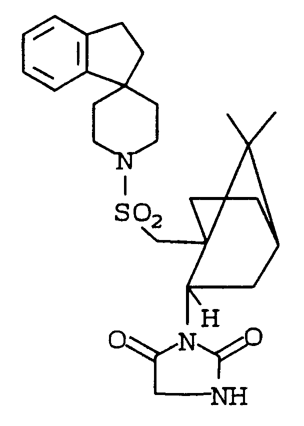

The procedure of Example 12 was carried out using the product of Example 1 [1.38 mmol], triethylamine [3.40 mmol], and substituting glycine methyl ester hydrochloride [1.54 mmol] for histidine methyl ester dihydrochloride. Chromatographic elution for column 1 was with 1% ether in CH₂Cl₂ and then with 4% methanol in CH₂Cl₂. For column 2, the elution was done with 99/1/0.1 of CH₂Cl₂/methanol/ammonium hydroxide. The title compound was obtained as a white solid from ether and dried in vacuo, overnight.
m.p.: 230°-239°C
NMR: Consistent with structure
HPLE: >92% pure
MS: M+H⁺=486 (FAB)
CHN:

| Calc'd for C₂₆H₃₅N₃O₄S·0.10 C₄H₁₀O·0.20 H₂O; | | | |
|---|---|---|---|
| | C, 63.84; | H, 7.39; | N, 8.46. |
| Found: | C, 63.77; | H, 7.39; | N, 8.50. |

### EXAMPLE 12

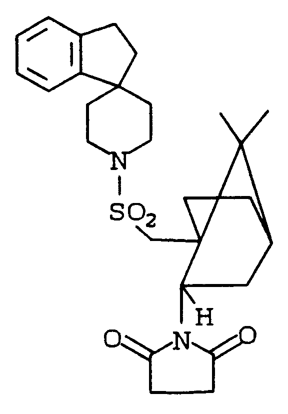

Succinic anhydride (12 mg, 0.12 mmols) and endo-(1S)-1'-(((2-amino-7,7-dimethylbicyclo-(2.2.1)-hept-1-yl)methyl)sulfonyl)spiro-(1H-indene-1,4'-piperidine) (50 mg, 0.12 mmols) were combined in a mixture of THF (1 mL) and methylene chloride (1 mL) and stirred at ambient temperature for eighteen hours. The solvents were removed under vacuum and the residue was treated with trifluoroacetic anhydride (1 mL) and toluene (2 mL), then heated to reflux for 15 minutes while the excess trifluoroacetic anhydride was allowed to boil out. The mixture was then cooled and evaporated to dryness in vacuo. The residue was chromatographed on silica gel (8" column, 0.5" diam.), eluted with 0.5% (100 mL) followed by 1% (100 mL) methanol in methylene chloride. The product fractions were combined and evaporated to dryness in vacuo. The residue was dissolved in ethyl acetate, diluted with hexane, and allowed to stand whereupon the title compound was deposited as a white solid. This material was filtered and dried in vacuo at 90° for eighteen hours.
m.p.: 228.5°-229.5°C
¹H-NMR: Consistent with structure, ca. 0.1 mol of ethyl acetate and ca. 0.05 mol of hexane observed
TLC: (2% MeOH in CH₂Cl₂) single component, R_{f}=0.66
MS: M+H⁺=485 (FAB)
CHN:

| Calc'd for C₂₇H₃₆N₂O₄S·0.10 C₄H₈O₂·0.05 C₆H₁₄; | | | |
|---|---|---|---|
| | C, 66.83; | H, 7.59; | N, 5.63. |
| Found: | C, 66.62; | H, 7.61; | N, 5.51. |

### EXAMPLE 13

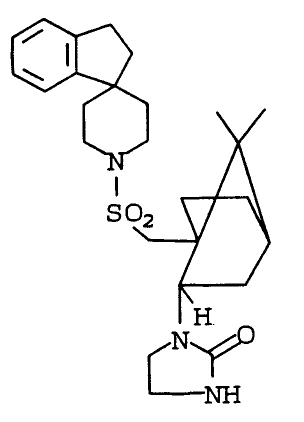

To a 0^{o}C solution of endo-(1S)-1'(((2-amino-7,7-dimethylbicyclo (2.2.1)-hept-1-yl)-methyl)-sulfonyl)spiro(1H-indan-1,4'-piperidine) (0.90 g; 2.2 mmol) and diisoprpylethylamine (DIEA) (0.47 mL; 2.7 mmol) in CHCl₃ (50 mL) was added iodoacetonitrile (0.38 grams; 2.3 mmol). The solution was stirred for 1 h at 0^{o}C and then for 18 h at ambient temperature. The mixture was extracted with aqueous NaHCO3 (2 x 25 mL), dried (MgSO₄), filtered, and the solvent was removed under reduced pressure. The residue was purified by pressurized silica gel column chromatography using 1:3 ethyl acetate-hexanes as eluant (TLC Rf = 0.30 in 1:3 ethyl acetate-hexanes; HPLC retention time = 9.30 min). The purified cyanomethylated amine (0.80 g; 1.8 mmol) was dissolved in 2-methoxyethanol (15 mL) and to the stirred solution was added Raney nickel alloy (2.5 grams) followed by 6N NaOH solution (2.0 mL, 12 mmol). The mixture was heated to 80^{o}C on a steam bath and then stirred at ambient temperature for 14 h. The catalyst was removed by filtration through Celite and washed with EtOAc. The filtrate solvents were removed under reduced pressure and the residue was taken up in CHCl₃ (50 mL) and washed with water (2 x 25 mL). The organic phase was dried (MgSO₄), filtered and concentrated under reduced pressure. The residue was purified by pressurized silica gel column chromatography using 92:8:0.8 CHCl₃:MeOH:NH₄OH as eluant (TLC Rf = 0.25 in 92:8:0.8 CHCl₃:MeOH:NH₄OH; HPLC retention time = 7.20 min; FAB mass spectrum m/z = 446). The purified diamine (0.51 g; 1.1 mmol) was dissolved in CHCl₃ and to the solution was added 1,1'-carbonyldiimidazole (0.19 g; 1.2 mmol). After the solution had been stirred for 1 h at ambient temperature, acetic acid (0.63 mL; 11 mmol) was added and the solution was refluxed for 6 h. The reaction was cooled and the solvent was removed under reduced pressure. The residue was dissolved in EtOAc (50 mL) and the solution was washed with 10% aqueous citric acid (25 mL), water (25 mL), and aqueous NaHCO₃ (25 mL). The organic phase was dried (MgSO₄), filtered, and the solvent was removed under reduced pressure. The residue was purified by pressurized silica gel column chromatography using 1:3 EtOAc:CHCl₃ as eluant. The title compound was obtained as a white foam from CHCl₃ (TLC Rf = 0.27 in 1:4 EtOAc:CHCl₃;
HPLC retention time = 10.67 min;
FAB mass spectrum m/z=472;

| calc for C₂₆H₃₇N₃O₃S-0.70 | | | |
|---|---|---|---|
| CHCl₃: | C, 57.76; | H, 6.84; | N, 7.75. |
| Found: | C, 57.84; | H, 6.82; | N, 7.42; |

¹H NMR (CDCl₃, 300 MHz) d 7.15-7.25 (m, 4H), 4.39 (ddd, J = 2.3, 5.3, 12.0 Hz, 1H) 1.05 (s, 3H), 1.00 (s, 3H)).
HPLC conditions: 12 cm C₁₈ reverse phase Vydac column; 15 min gradient 95:5 to 0:100 A:B (A = H₂O containing 0.1% TFA, B = CH₃CN containing 0.1% TFA), flow rate = 2.0 mL/min, detection at 215 nm.

### EXAMPLE 14

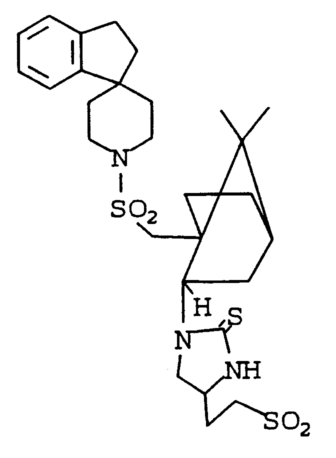

2-Amino-[1-[[(2,3-dihydrospiro[1H-indene-1,4'-piperidin]-1'-yl)sulfonyl]methyl]-7,7-dimethylbicyclo [2.2.1]hept-2-yl]-4-(methylsulfonyl)-but-1-ylamine (250 mg, 0.425 mmole) and thiocarbonyl- diimidazole (76 mg, 0.425 mmole) were combined with 500 mg of anhydrous cesium carbonate in 12 ml of dry N,N'-dimethylformamide at room temperature. The orange suspension was stirred for 2 hours, filtered, and concentrated under reduced pressure. The residue was partitioned between ethyl acetate (100 ml) and sodium bicarbonate solution. The phases were separated and the organic phase was washed with saturated sodium bicarbonate solution (3 X 40 ml) and brine, then dried (sodium sulfate) and concentrated. The crude product was (250 mg) was obtained as an oil which crystallized on standing in methanol:
NMR: Consistent with structure and verifies presence of solvent;
HPLC: > 97% pure at 214 nm;
FAB MS: 594 (M+ + H);

| Elem. Anal. calc'd for C₂₉H₄₃N₃O₄S₃·1.05 CH₃OH·0.25H₂O: | | | |
|---|---|---|---|
| Calc'd: | C, 57.10; | H, 7.61; | N, 6.65. |
| Found: | C, 57.11; | H, 7.21; | N, 6.28. |

### EXAMPLE 15

### 1-[1-[[(2,3-Dihydrospiro[1H-indene-1,4'-piperidin]-1'-yl)sulfonyl]-methyl]-7,7-dimethylbicyclo[2.2.1]hept-2-yl]-2,5-dioxo-3-pyrrolidineacetic acid

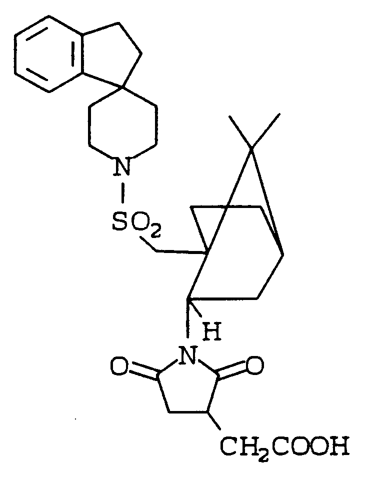

2-Carboxymethylsuccinic anhydride (3-carboxymethyltetrahydrofuran-2,5-dione) was prepared from tricarballylic acid as described in J. Org. Chem. 46 2866 (1981). 2-Carboxymethylsuccinic anhydride (0.93g, 5.88 mmols) and endo-(1S)-1'-(((2-amino-7,7-dimethylbicyclo-(2.2.1)-hept-1-yl)methyl)sulfonyl)spiro(1H-indene-1,4'-piperidine) (2.4g, 5.97 mmols) were combined in DMF (20 mL) and stirred at ambient temperature for eighteen hours. The DMF was removed under vacuum and the residue was treated with 1N HCl and extracted with methylene chloride. The methylene chloride layers were combined, dried over sodium sulfate, filtered, and evaporated to dryness in vacuo. The residue was treated with toluene (100 mL) and trifluoroacetic anhydride (5mL), and the resulting mixture was heated to reflux for 2-4 min while the excess trifluoroacetic anhydride was allowed to boil out. Reflux was continued for 10 min, and the mixture was then cooled and evaporated to dryness in vacuo. The residue was chromatographed on silica gel (10" column, 2"diam.), eluted with 200:10:1:1 CH₂Cl₂:MeOH:HoAc:H₂O. The product obtained by evaporation of the eluate was rechromatographed on silica gel twice, once eluted with 1L each of 1000:10:1:1, 500:10:1:1, and 330:10:1:1 CH₂Cl₂:MeOH:HoAc:H₂O, and the second time with 600:10:1:1 of the same solvents. The combined product fractions were evaporated to dryness in vacuo, treated with ether and re-evaporated 3 times, then treated with hexane and evaporated to obtain the title compound as a solid which was dried in vacuo at 400°C for eighteen hours.
M.P. 80-100°C (foam;indistinct)
HPLC: 100%
¹H-NMR: Consistent with structure, ca. 0.05 mol of DMF and ca. 0.18 mol of hexane observed.
TLC: (490:10:1:1 CH₂Cl₂:MeOH:HoAc:H₂O) single component, R_{f} = 0.25.
M.S.: (FAB) M+H @ 543

| Analysis for C₂₉H₃₈N₂O₆.0.05 C₃H₇NO.0.18 C₆H₁₄.0.3 H₂O: | | | |
|---|---|---|---|
| Calc'd: | C, 64.00; | H, 7.37; | N, 5.06 |
| Found: | C, 64.01; | H, 7.30; | N, 5.12. |

### EXAMPLE 16

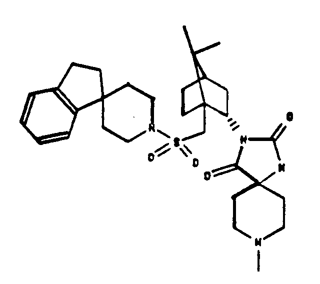

To a 0°C stirred solution of p-nitrophenyl chloroformate (1.37 g; 6.8 mmol) in CHCl₃ (100 mL) was added DIEA (1.18 ml; 12.4 mmol) and the product of Example A (2.5 g; 6.2 mmol). The solution was stirrred at 0°C for 1 h and then at ambient temperature for 14 h. The reaction mixture was concentrated under reduced pressure, the residue was dissolved in CHCl₃ (100 mL) and washed with 5% aqueous HCl (2 x 50 mL) and aqueous NaHCO₃ (100 mL). The organic phase was dried (MgSO₄), filtered, and the solvent was removed under reduced pressure. The urethane was obtained as a white foam.
- TLC:: R_{f} 0.35 (1:3 EtOAc:hexanes)
- HPLC (method A):: retention time 12.3 min

To a 0°C stirred solution of the p-nitrophenyl urethane (2.8 g; 5.0mmol) in DMF (20 mL) was added methyl 1-methyl-4-amino-4-piperidine carboxylate hydrochloride (1.04 gm, 5 mmol) and DIEA (0.87 ml, 5 mmol). The solution was stirred for 2 hours at ambient temperature. The reaction mixture was concentrated under reduced pressure, the residue was dissolved in CHCl₃ (100 mL) and washed with 5% aqueous HCl (2 x 50 mL) and 10 % aqueous Na₂CO₃ (5 x 100 mL). The organic phase was dried (MgSO₄), filtered, and the solvent was removed under reduced pressure. The urea was obtained as a foam which was crystallized from EtOAc ( 1.14 gm, 2 mmol).

| Anal: (C₃₁H₄₄N₄O₄S)^{.}1.85 H₂O | | | |
|---|---|---|---|
| Calc. | C 60.61 | H 8.22 | N 8.84 |
| Found | C 60.58 | H 8.02 | N 8.80 |

- TLC:: R_{f} 0.2 (95: 5: 0.5 CHCl₃: MeOH: NH₃OH)
- HPLC (method A):: retention time 9.17 min
- FAB MS:: m/z 601 (M⁺ + H)

To a 0°C stirred solution of the urea (1.0 gm, 1.67 mmol) in MeOH ( 50 mL) was added in small portions NaH (dry powder) (0.125 gm, 5 mmol). The solution was stirred for 2 hours. The reaction mixture was neutralized with acetic acid and evaporated under reduced pressure. The residue was dissolved in CHCl₃ (100 mL) and washed with 5% aqueous HCl (2 x 50 mL) and aqueous NaHCO₃ (100 mL). The organic phase was dried (MgSO₄), filtered, and the solvent was removed under reduced pressure. The title compound was obtained as a foam which was precipitated from EtOAc/ hexanes ( 0.260 gm, 0.5 mmol).

| Anal: (C₃₁H₄₄N₄O₄S)^{.}0.3 EtOAc | | | |
|---|---|---|---|
| Calc. | C 64.98 | H 7.86 | N 9.41 |
| Found | C 64.65 | H 7.76 | N 9.46 |

- TLC:: R_{f} 0.35 (95:5:0.5 CHCl₃:MeOH:NH₄OH)
- HPLC (method A):: retention time 10.17 min
- FAB MS:: m/z 569 (M⁺ + H)
- ¹H NMR (300 MHz, CDCl₃):: δ 7.15-7.25 (m, 4H), 5.8 (s, 1H), 4.49 (m, 1H), 2.3 (s, 3H), 1.02 (s, 3H), 0.97 (s, 3H)

### EXAMPLE 17

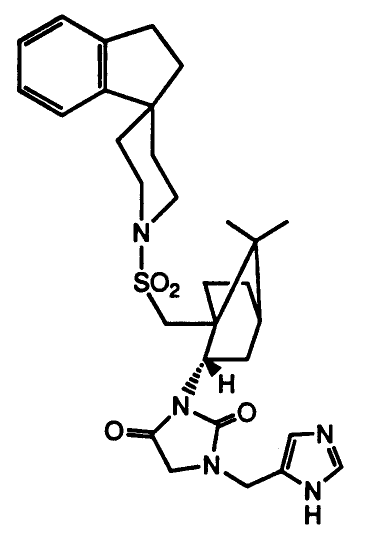

To a 0°C solution of the unsubstituted hydantoin product of Example 11 (1.50 g; 3.09 mmol) and 4-chloromethyl-1-(triphenyl)methylimidazole (1.39 g; 3.87 mmol) in dry THF (60 mL) under an atmosphere of argon was added NaH (154 mg of a 60% suspension in mineral oil; 3.86 mmol). The mixture was stirred at 0°C for 1 h, and then at ambient temperature for 24 h. Several drops of acetic acid were added and the mixture was concentrated under reduced pressure. The residue was dissolved in EtOAc (100 mL) and washed with aqueous NaHCO₃ (2 x 50 mL). The organic phase was dried (MgSO₄), filtered and concentrated under reduced pressure. The residue was purified by pressurized silica gel column chromatography using 1:1 EtOAc:CHCl₃ as eluant. The product (1.40 g; 1.73 mmol) was heated in 10 mL of MeOH containing 10 mL of 6N HCl at 60°C for 6 h. The solvents were removed under reduced pressure and the residue was dissolved in CHCl₃ (100 mL) and washed with aqueous NaHCO₃ (2 x 50 mL). The organic phase was dried (MgSO₄), filtered, and concentrated under reduced pressure. The residue was purified by pressurized silica gel column chromatography using 95:5:0.5 CHCl₃:MeOH:NH₄OH as eluant. The purified product was dissolved in MeOH containing 3 equivalents of 6 N HCl and the solvent was removed under reduced pressure. The residue was taken up in water-dioxane and lyophilized to give the HCl salt of title compound as a white powder.

| Anal: (C₃₀H₃₉N₅O₄S)^{.}2.05 HCl.0.55 H₂O | | | |
|---|---|---|---|
| Calc. | C, 57.40; | H, 6.53; | N, 10.77. |
| Found | C, 57.44; | H, 6.53; | N, 10.41. |

- TLC:: R_{f} 0.29 (95:5:0.5 CHCl₃:MeOH:NH₄OH)
- HPLC (method A):: retention time 9.43 min
- FAB MS:: m/z 566 (M⁺ + H)
- ¹H NMR (300 MHz, CDCl₃):: δ 8.95 (s, 1H), 7.40 (s, 1H), 7.15-7.25 (m, 4H), 4.75 (m, 2H), 4.55 (m, 1H), 1.03 (s, 3H), 0.97 (s, 3H)

### EXAMPLE 18

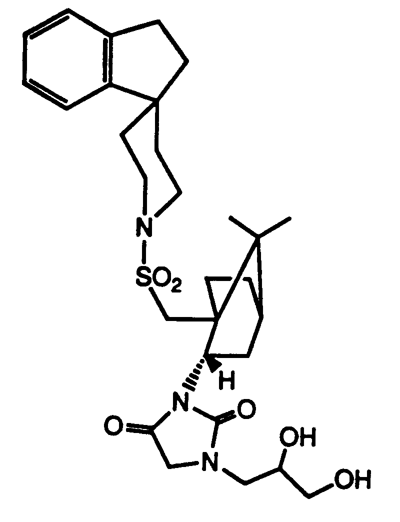

To a stirred solution of the hydantoin (FRANK: L-369,156) (150 mg; 0.309 mmol) in a mixture of 2:1 allyl bromide:tetrahydrofuran (30 mL) was added sodium hydride (12 mg; 60% dispersion in oil). The temperature was then increased to reflux. After 1 hr the solution was cooled, then concentrated. Purification by flash chromatography (5% methanol in methylene chloride) provided the intermediate allyl derivative (158 mg).

The allyl hydantoin described above 105 mg; 0.20 mmol) was dissolved in a solution of 1:1 pyridine:toluene (12 mL). While stirring at room temperature, osmium tetraoxide (51 mg; 0.20 mmol) was added. After 8 hr 10 mL of a saturated aqueous solution of sodium bisulfite was added. The solution was allowed to stir for 1 hr, then diluted with ethyl acetate (50 mL). The ethyl acetate was separated, dried over sodium sulfate, then concentrated. Purification of the residue by flash chromatography (10% methanol in methylene chloride) afforded the title compound (39 mg; 35%).

| Anal: (C₂₉H₄₁N₃O₆S)^{.}0.56 H2) | | | |
|---|---|---|---|
| Calc. | C, 61.13; | H, 7.45; | N, 7.37 |
| Found | C, 61.15; | H, 7.55; | N, 7.15 |

- HPLC:: (Vydac C18 Column; gradient from 95/5 to 0/100 H₂0/CH₃CN with 0.1% TFA. 15 min. gradient, flow rate = 1.5 ml/min.) Rₜ = 12.12 min. Purity = 96%
- ¹HNMR:: Consistent with structure
- FABMS:: m/z = 560 (M⁺ + H)

### EXAMPLE 19

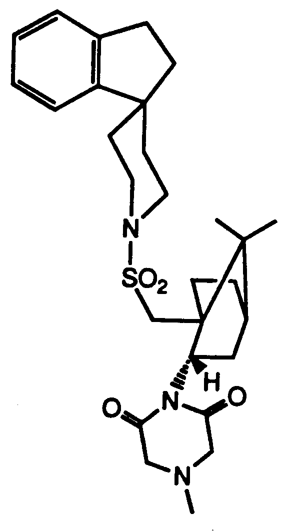

To a stirred solution of N-methyliminodiacetic acid (220 mg; 1.50 mmol) in DMF (10 mL) was added DIEA (0.575 mL; 3.30 mmol) and BOP (665 mg; 1.50 mmol). The mixture was stirred at ambient temperature for 24 h, and then the product of Example A (500 mg; 1.24 mmol) was added. The mixture was strirred at ambient temperature for 24 h and then the solvent was removed under reduced pressure. The residue was dissolved in EtOAc (50 mL) and washed with 10% aqueous citric acid (20 mL) and water (10 mL). The organic phase was dried (MgSO₄), filtered, and the solvent was removed under reduced pressure. The residue was purified by pressurized silica gel column chromatography using a gradient elution of 5-10% MeOH-CHCl₃. The purified monoacid, monoamide was obtained as a white foam.
- TLC:: R_{f} 0.40 (90:10 CHCl₃:MeOH)
- HPLC (method A):: retention time 9.03 min
- FAB MS:: m/z 532 (M⁺ + H)

The purified monoacid, monoamide (150 mg; 0.282 mmol) was heated to reflux in a solution of THF (5 mL) and acetic anhydride (1 mL) for 14 h. The solvents were removed under reduced pressure and the residue was purified by pressurized silica gel column chromatography using 1:4 EtOAc-hexanes as eluant. The title compound was obtained as a white foam from ether.

| Anal: (C₂₈H₃₉N₃O₄S)^{.}0.2 ether^{.}0.1 H₂O | | | |
|---|---|---|---|
| Calc. | C, 65.23; | H, 7.83; | N, 7.92; |
| Found | C, 65.10; | H, 7.99; | N, 7.95; |

- TLC:: R_{f} 0.29 (1:2 EtOAc:hexanes)
- HPLC (method A):: retention time 10.57 min
- FAB MS:: m/z 514 (M⁺ + H)
- ¹H NMR (300 MHz, CDCl₃):: δ 7.10-7.25 (m, 4H), 5.20 (ddd, J = 2.2, 5.9, 12.1 Hz, 1H), 3.40 (s, 3H), 2.37 (s, 3H), 1.06 (s, 3H), 0.95 (s, 3H)

### EXAMPLE 20

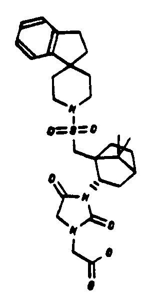

(1S)-1'-(((2-endo-Amino-7,7-dimethylbicyclo-(2.2.1) hept-1-yl)methyl) sulfonyl)spiro(1H-indane-1,4'-piperidine) (1.5 g, 3.7 mmole), tert-butylbromo acetate (0.8 g, 4.1 mmole), and crushed potassium carbonate (0.57 g, 4.1 mmole) were combined in 80 ml of absolute ethanol and heated at reflux for 12 hours. The reaction mixture was cooled, filtered, and rotoevaporated under reduced pressure. The residual material was partitioned between ethyl acetate and water. The phases were separated and the organic layer was washed in succession with saturated sodium bicarbonate solution and brine, then dried (sodium sulfate), and concentrated to give a semi-solid. The crude product was crystallized from ethyl acetate to give 0.85 g of (1S)-1'-(((2-endo-tert-butyloxycarbonylmethylamino-7,7-dimethylbicyclo-(2.2.1) hept-1-yl)methyl)sulfonyl)spiro(1H-indane-1,4'-piperidine). Concentration of the mother liquors afforded an additional 0.99 g of material.

A solution of 40 ml of methylene chloride containing 0.41 ml of triethylamine and 0.97 g of (1S)-1'-(((2-endo-tert-butyloxycarbonylmethylamino-7,7-dimethylbicyclo(2.2.1) hept-1-yl)methyl) sulfonyl) spiro(1H-indane-1,4'-piperidine) was stirred magnetically in an ice bath and treated in one portion with 0.24 ml of bromoacetylbromide. After 1 hour, an additional equivalent each of bromoacetylbromide and triethylamine were added and the reaction mixture was stirred at ambient temperature overnight. The reation mixture was diluted with methylene chloride and washed in succession with sodium bicarbonate solution, 10% citric acid solution, and brine. The dried extracts were concentrated and the residual material was flash chromatographed on silica gel (15% ethyl acetatehexane) to give 0.74 g of 2-tert-butyloxycarbonylmethylamino-N-[1-[[(2,3-dihydrospiro[1H-indane-1,4'-piperidin]-1'-yl)sulfonyl]methyl]-7,7-dimethylbicyclo[2.2.1]hept-2-yl]-bromoacetamide.

A continuous stream of ammonia gas was passed for 10 minutes into an ice cold solution of methanol (32 ml) containing 0.64 g (1.0 mmole) of 2-tert-butyloxycarbonylmethylamino-N-[1-[[(2,3-dihydrospiro[1H-indane-1,4'-piperidin]-1'-yl)sulfonyl]methyl]-7,7-dimethylbicyclo[2.2.1]hept-2-yl)-bromoacetamide. The reaction mixture was warmed to room temperature and stirred for 1 hour. All volatile components were removed under reduced pressure to give a semi-solid which was partitioned between ethyl acetate and water. The organic phase was washed with water (3X) and brine, then dried (sodium sulfate) and concentrated. Triturated of the residual material with ether gave 0.32 g of the title compound as an off-white solid: m.p. >220°C;
NMR: Consistent with structure:
HPLC: > 99% pure at 214 nm;
FAB MS: 500 (M⁺ + H);

| Elem. Anal. calc'd for C₂₇H₃₇N₃O₄S·0.25 H₂O: | | | |
|---|---|---|---|
| Calc'd: | C, 64.31; | H, 7.51; | N, 8.34. |
| Found: | C, 64.32; | H, 7.34; | N, 8.14. |

### EXAMPLE 21

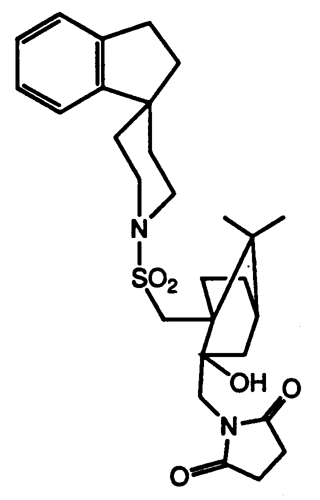

To an ice cold suspension trimethylsulfoxonium iodide (610 mg, 2.77 mmole) in 10 ml of dry tetrahydrofuran was added 1.8 ml of 1.6M n-butyllithium under nitrogen. After addition was complete the resulting reaction mixture was stirred at ambient temperature for 2 hours, re-cooled to 0°C, and treated with a tetrahydrofuran solution (6 ml) containing 620 mg (1.55 mmole) of (1S)-1'-(((7,7-dimethyl-2-oxobicyclo[2.2.1]hept-1-yl)methyl)sulfonyl) spiro(1H-indene-1,4'-piperidine). The reaction mixture was then stirred at ambient temperature overnight. The reaction mixture was concentrated under reduced pressure to a volume of 6 ml and chromatographed on silica gel (hexane-ethyl acetate, 4:1) separating unreacted starting material and affording 390 mg of (1S)-1'-(((7,7-dimethyl-2-oxiranebicyclo[2.2.1]hept-1-yl)methyl)sulfonyl) spiro-(1H-indene-1,4'-piperidine).

To a suspension of 1.7 mmole of sodium hydride in 1.7 ml of dry N,N'-dimethylformamide was added 0.18 mmole of succinimide. After stirring for 15 minutes the reaction mixture became homogeneous and 70 mg (0.17 mmole) of (1S)-1'-(((7,7-dimethyl-2-oxiranebicyclo[2.2.1]hept-1-yl)methyl)sulfonyl)spiro(1H-indene-1,4'-piperidine) was added. The reaction mixture was heated at 150°C for 4 hours, then cooled to room temperature, and diluted with ethyl acetate. The organic phase was washed with water and brine, then dried, and concentrated to give 92 mg of crude product. Flash column chromatography on silica gel (30% ethyl acetate-hexane elution) of the crude reaction product afforded the title compound in analytically pure form as a white solid: m.p. 111-115°C;
NMR: Consistent with structure:
HPLC: > 99% pure at 214 nm;
FAB MS: 513 (M⁺ + H), 621 (M⁺ + thioglycerol);

| Elem. Anal. calc'd for C₂₈H₃₆N₂O₅S·0.75 H₂O: | | | |
|---|---|---|---|
| Calc'd: | C, 63.90; | H, 7.20; | N, 5.32. |
| Found: | C, 63.86; | H, 7.14; | N, 5.10. |

### EXAMPLE 22

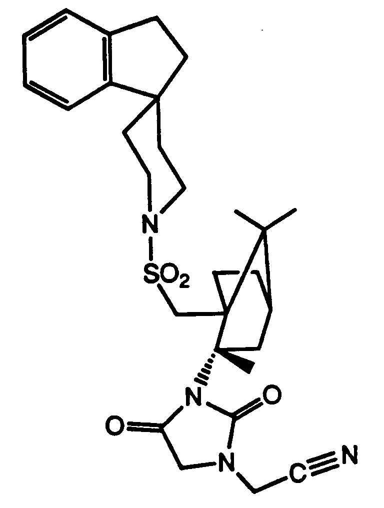

To a 0°C solution of the unsubstituted hydantoin product of Example 11 (1.50 g; 3.09 mmol) and iodoacetonitrile (1.03 g; 6.18 mmol) in dry THF (30 mL) under an atmosphere of argon was added NaH (185 mg of a 60% suspension in mineral oil; 4.64 mmol). The mixture was stirred at 0°C for 1 h, and then at ambient temperature for 6 h. The reaction was cooled to 0°C and more iodoacetonitrile (0.52 g; 3.1 mmol) and NaH (124 mg of a 60% suspension in mineral oil; 3.1 mmol) were added. The mixture was stirred at 0°C for 1 h, and then at ambient temperature for 14 h. Several drops of acetic acid were added and the dark brown mixture was concentrated under reduced pressure. The residue was dissolved in EtOAc (100 mL) and washed with aqueous NaHCO₃ (2 x 50 mL). The organic phase was dried (MgSO₄), filtered and concentrated under reduced pressure. The residue was purified by pressurized silica gel column chromatography using 7:3 hexane:EtOAc as eluant, and then by preparative reverse phase HPLC using a water-acetonitrile gradient containing 0.1% TFA. The title compound was obtained as a lyophilized powder.

| Anal: (C₂₈H₃₆N₄O₄S)^{.}0.35 TFA^{.}0.25 H₂O | | | |
|---|---|---|---|
| Calc. | C, 60.57; | H, 6.53; | N, 9.85. |
| Found: | C, 60.51; | H, 6.44; | N,10.22 |

- TLC:: R_{f} 0.43 (3:2 hexane:EtOAc)
- HPLC (method A):: retention time 11.39 min
- FAB MS:: m/z 525 (M⁺ + H)
- ¹H NMR (300 MHz, CDCl₃):: δ 7.1-7.3 (m, 4H), 4.56 (m, 1H), 4.35 (AB quartet, J = 18 Hz, 2H), 3.95 (AB quartet, J = 16 Hz, 2H), 1.06 (s, 3H), 0.97 (s, 3H)

### EXAMPLE 23

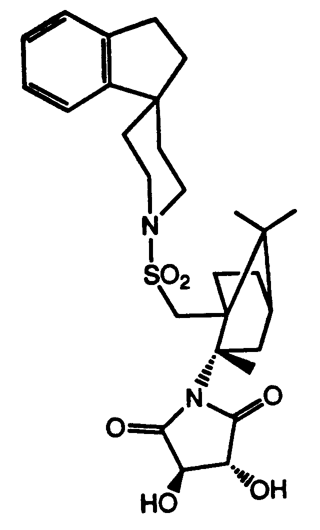

To a stirred solution of endo-(1S)-1'(((2-amino-7,7-dimethylbicyclo(2.2.1)-hept-1-yl)-methyl)sulfonyl)spiro(1H-indan-1,4'-piperidine (526 mg; 1.31 mmol) in methylene chloride (20 mL) was added diacetyl-L-tartaric anhydride (312 mg; 1.44 mmol), followed by diisopropylethyl amine (0.251 mL; 1.44 mmol). After 18 hr the solution was concentrated, then partitioned between ethyl acetate (200 mL) and 1M HCl (200 mL). The ethyl acetate layer was washed with additional water (2 x 200 mL), then dried over sodium sulfate and concentrated. Partial purification by flash chromatography (10% methanol in methylene chloride) afforded material that was dissolved in methylene chloride (20mL) and treated with thionyl chloride (0.096 mL; 1.31 mmol). After stirring at room temperature for 18 hr, the solution was concentrated. The intermediate diacetate was obtained by purification of the residue by flash chromatography (10% methanol in methylene chloride).

The diacetate described above (1 g; 1.66 mmol) was dissolved in a solution of 3:1 tetrahydrofuran:water (40 mL) then cooled to 0°C. A solution of 30% hydrogen peroxide (0.832 mL; 6.64 mmol) was added followed by lithium hydroxide (80 mg; 3.32 mmol). After stirring at 0°C for 30 min the solution was concentrated. The title compound (492 mg; 73%) was obtained through purification of the residue by flash chromatography (10% methanol in methylene chloride).

| Anal: (C₂₇H₃₆N₂O₆S)^{.}0.35 H₂O | | | |
|---|---|---|---|
| Calc. | C, 62.01; | H, 7.07; | N, 5.36 |
| Found | C, 62.00; | H, 6.86; | N, 5.47 |

- HPLC:: (Vydac C18 Column; gradient from 95/5 to 0/100 H₂0/CH₃CN with 0.1% TFA. 15 min. gradient, flow rate = 1.5 ml/min.) Rₜ = 12.5 min. Purity = 100%
- ¹HNMR:: Consistent with structure
- FABMS:: m/z = 517 (M⁺ + H)

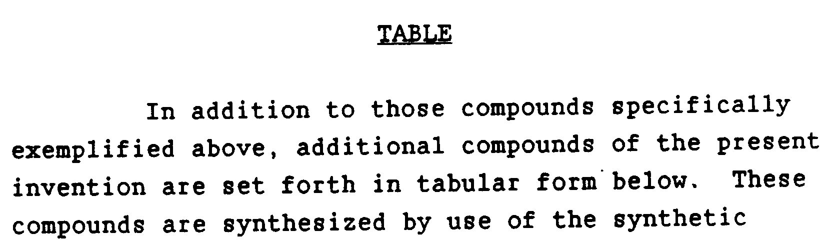

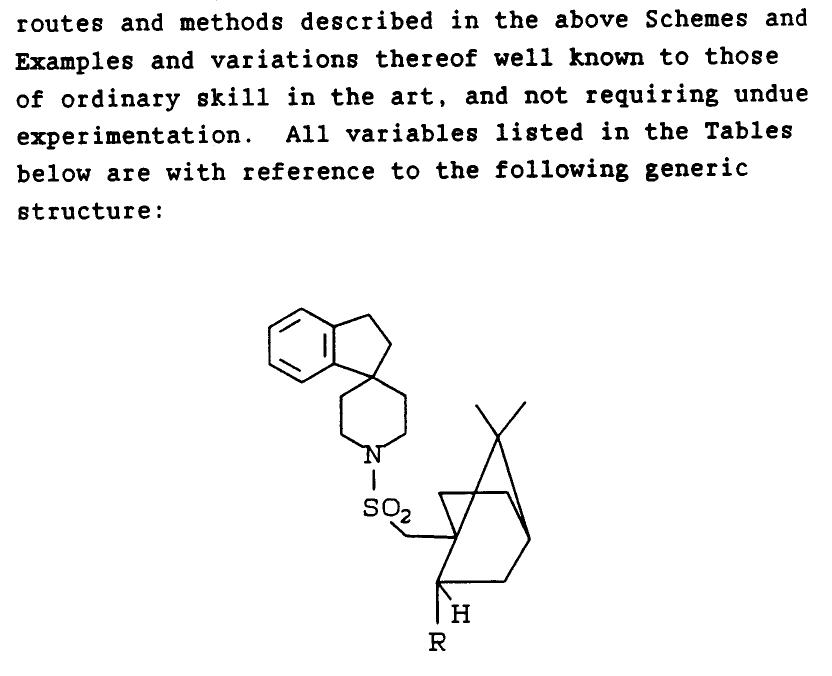

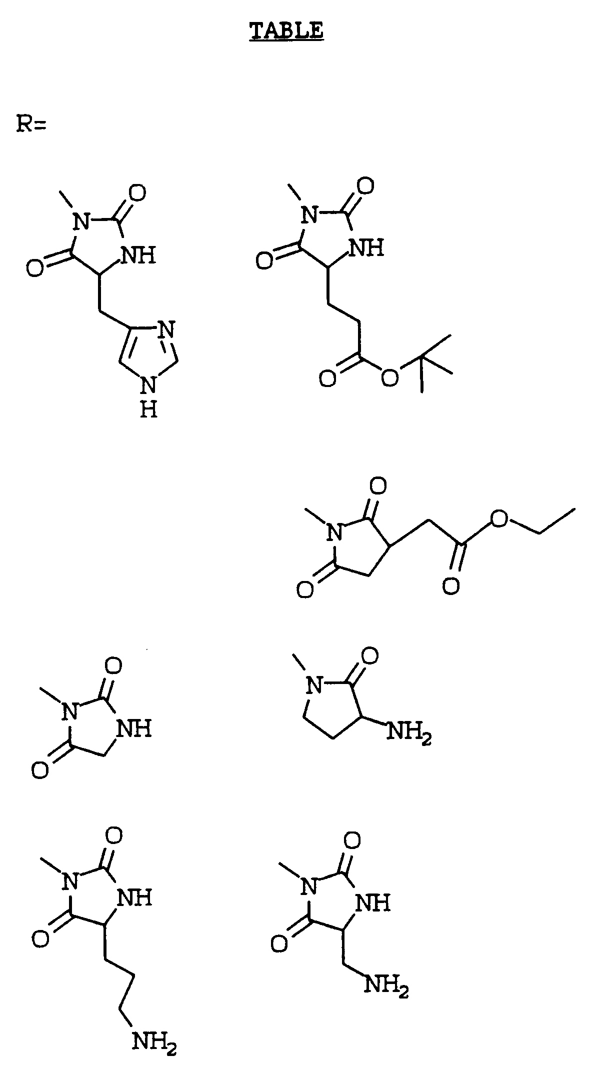

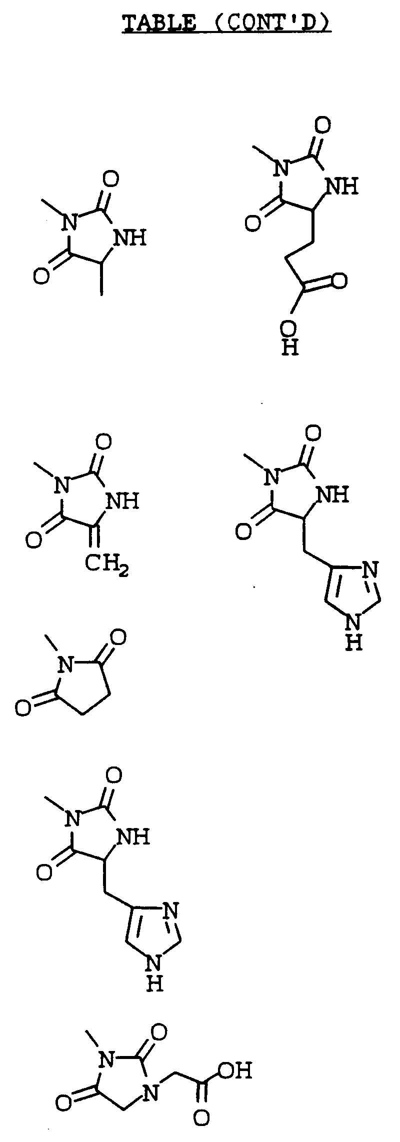

### RADIOLIGAND BINDING ASSAYS

The high affinity binding of [³H] Oxytocin (OT)([tyrosyl, 3,5-[³H]OT; 30-60 Ci/mmol; New England Nuclear. Boston, MA) to uterine OT receptors was based on an assay* using a crude membrane preparation of uteri taken from diethylstilbestrol dipropionate (DES)-treated (0.3 mg/kg, ip; 18-24) rats. Competition studies were conducted at equilibrium (60 minutes; 22°C) using 1 nM[³H]OT in the following assay buffer: 50 mM Tris-HCl, 5 mM MgCl₂, and 0.1% BSA, pH 7.4. Nonspecific binding (10% of the total binding) was determined using 1µM unlabeled OT and the binding reaction was terminated by filtration through glass fiber filters using a cell harvester (model 7019, Skatron, Inc., Sterling, VA). IC₅₀ (the concentration of tested compound that inhibits 50% of OT) was reported, unless otherwise noted.
* Fuchs, A-R; Fuchs, F; Soloff, MS. 1985 J. Clin. Endocrinol. Metab. 60:37.

The measurement of [³H]Vasopressin (AVP) ([phenylalanyl-3,4,5-³H]AVP; 80-90 Ci/mmol; New England Nuclear)binding to a crude membrane preparation of male rat liver (AVP-V₁ sites) or kidney medulla (AVP-V₂ sites) was determined according to the method of Butlen, et al.** Competition assays were conducted at equilibrium (30 minutes at 30°C) using 1 nM [³H]AVP (liver) or 2 nM [³H]AVP (kidney) in the following assay buffer: 100 mM Tris-HCl, 5 mM MgCl₂, 0.1% BSA, 50 µM phenylmethylsulfonylfluoride, and 50 µg/ml bactracin, pH 8.0. Nonspecific binding (5-10% of the total binding) was determined using 10 µM unlabeled AVP, and the binding reaction was terminated by filtration as described above for the [³H]OT binding assay.
** Butlen, D; Guillon, G; Rajerison, R.M.; Jard, S; Sawyer, W.H.; Manning, M. 1978 Mol Pharmacol 14:1006.

Kᵢ; values were obtained for each compound from three to six separate determinations of the IC₅₀ values (Kᵢ = IC₅₀/1 + c/K_{d})*** using K_{d} values obtained from saturation binding assay: [³H]OT (uterus), 0.7 nM; [³H]AVP (liver), 0.4 nM; [³H] (kidney), 1.4 nM.
*** Cheng, Y-C; Prusoff, W.H.; 1973 Biochem Pharmacol 22:3099

Effective dosages other than the preferred dosages as set forth hereinabove may be applicable as a consequence of variations in the responsiveness of the mammal being treated for prevention of preterm labor, or for other indications for the compounds of the invention indicated above. Likewise, the specific pharmacological responses observed may vary according to and depending upon the particular active compound selected or whether there are present pharmaceutical carriers, as well as the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of the formula 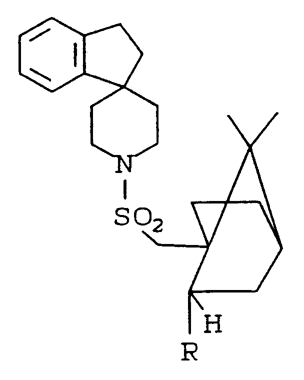 and the pharmaceutically acceptable salts thereof,
wherein
R is
Het-R', wherein
Het is
substituted saturated or unsaturated 5 or 6 membered heterocyclic rings containing 1, 2 or 3 heteroatoms, wherein said heteroatoms are N, O or S;
R' is independently one or more of hydrogen, oxo, thiono, amino or unsubstituted or substituted alkyl, where said alkyl substituent is independently one or more of hydroxyl, amino, oxo, sulfonyl, alkylsulfonyl, carboxyl, phenyl, indole, alkoxycarbonyl, carbonylamino, guanidino, alkoxycarbonylamino, imidazole, imidazolylalkylaminocarbonyl, pyrrolidine,tetrazolylaminocarbonyl, tetrazolylalkylcarbonylamino, alkylaminoalkylaminocarbonyl and dialkylaminoalkylaminocarbonyl.

2. A compound of the formula 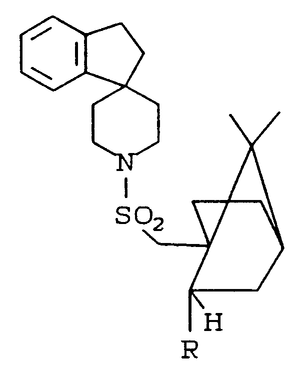 and the pharmaceutically acceptable salts thereof,
wherein
R is
Het-R', wherein
Het is
substituted 5 membered heterocyclic rings containing 1 or 2 heteroatoms, wherein said heteroatoms are N;
R' is independently one or more of hydrogen, oxo, amino or unsubstituted or substituted alkyl, where said alkyl substituent is independently one or more of hydroxyl, amino, oxo, sulfonyl, alkylsulfonyl, carboxyl, phenyl, indole, alkoxycarbonyl, alkoxycarbonylamino, imidazole or pyrrolidine.

3. The compound as claimed in Claim 1, of the formula

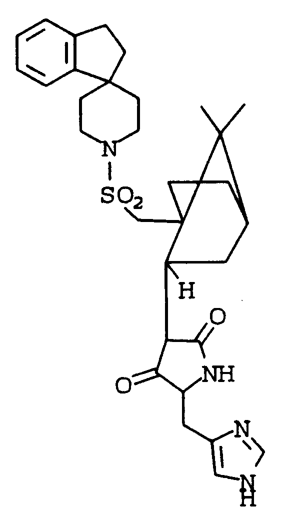

4. A pharmaceutical composition comprising a pharmaceutically acceptable carrier and a pharmacologically effective amount of the compound as claimed in Claim 1, sufficient to antagonize oxytocin from binding to its receptor site.

5. The use of a compound as claimed in Claim 1 for the manufacture of a medicament for antagonizing oxytocin from binding to its receptor site in a mammal.

6. A pharmaceutical composition comprising a pharmaceutically acceptable carrier and a pharmacologically effective amount of the compound as claimed in Claim 1 sufficient to prevent preterm labor in a mammal in need thereof.

7. The use of a compound as claimed in Claim 1 for the manufacture of a medicament for preventing preterm labor in a mammal.

8. A pharmaceutical composition comprising a pharmaceutically acceptable carrier and a pharmacologically effective amount of the compound as claimed in Claim 1, sufficient to stop labor preparatory to cesarian delivery.

9. The use of a compound as claimed in Claim 1 for the manufacture of a medicament for stopping labor preparatory to cesarian delivery in a mammal.

10. A pharmaceutical composition comprising a pharmaceutically acceptable carrier and a pharmacologically effective amount of the compound as claimed in Claim 1, sufficient to treat dysmenorrhea.

11. The use of a compound as claimed in Claim 1 for the manufacture of a medicament for treating dysmenorrhea in a mammal.

12. The use of a compound as claimed in Claim 1 for the manufacture of a medicament for antagonizing vasopressin from binding to its receptor site in a mammal.

## Patentansprüche

1. Eine Verbindung der Formel 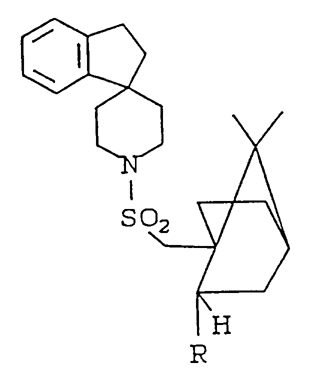 und die pharmazeutisch annehmbaren Salze davon, worin
R Het-R' ist, worin
Het substituierte gesättigte oder ungesättigte 5- oder 6gliedrige heterocyclische Ringe ist, die 1, 2 oder 3 Heteroatome enthalten, worin die Heteroatome N, O oder S sind,
R' unabhängig einer oder mehrere der Reste Wasserstoff, Oxo, Thiono, Amino oder unsubstituiertes oder substituiertes Alkyl ist, worin der Alkylsubstituent unabhängig einer oder mehrere der Reste Hydroxyl, Amino, Oxo, Sulfonyl, Alkylsulfonyl, Carboxyl, Phenyl, Indol, Alkoxycarbonyl, Carbonylamino, Guanidino, Alkoxycarbonylamino, Imidazol, Imidazolylalkylaminocarbonyl, Pyrrolidin, Tetrazolylaminocarbonyl, Tetrazolylalkylcarbonylamino, Alkylaminoalkylaminocarbonyl und Dialkylaminoalkylaminocarbonyl ist.

2. Ein Verbindung der Formel 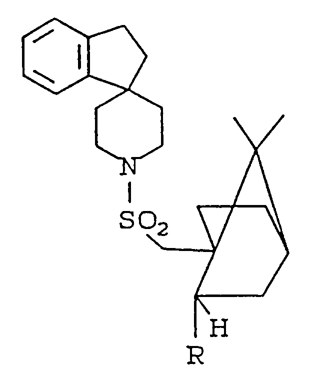 und die pharmazeutisch annehmbaren Salze davon, worin
R Het-R' ist, worin
Het substituierte 5gliedrige heterocyclische Ringe ist, die 1 oder 2 Heteroatome enthalten, worin die Heteroatome N sind,
R' unabhängig einer oder mehrere der Reste Wasserstoff, Oxo, Amino oder unsubstituiertes oder substituiertes Alkyl ist, worin der Alkylsubstituent unabhängig einer oder mehrere der Reste Hydroxyl, Amino, Oxo, Sulfonyl, Alkylsulfonyl, Carboxyl, Phenyl, Indol, Alkoxycarbonyl, Alkoxycarbonylamino, Imidazol oder Pyrrolidin ist.

3. Die wie in Anspruch 1 beanspruchte Verbindung der Formel

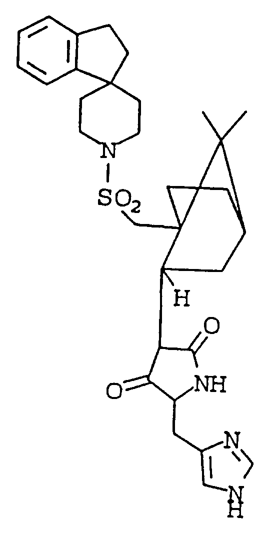

4. Eine pharmazeutische Zusammensetzung, umfassend einen pharmazeutisch annehmbaren Träger und eine pharmakologisch wirksame Menge der wie in Anspruch 1 beanspruchten Verbindung, die ausreicht, um die Bindung von Oxytozin an seine Rezeptorstelle zu antagonisieren.

5. Die Verwendung einer wie in Anspruch 1 beanspruchten Verbindung zur Herstellung eines Medikaments zur Antagonisierung der Bindung von Oxytozin an seine Rezeptorstelle in einem Säugetier.

6. Eine pharmazeutische Zusammensetzung, umfassend einen pharmazeutisch annehmbaren Träger und eine pharmakologisch wirksame Menge der wie in Anspruch 1 beanspruchten Verbindung, die ausreicht, um vorzeitige Wehen bei einem Säugetier, das dies bedarf, zu verhindern.

7. Die Verwendung einer wie in Anspruch 1 beanspruchten Verbindung zur Herstellung eines Medikaments zur Verhinderung vorzeitiger Wehen bei einem Säugetier.

8. Eine pharmazeutische Zusammensetzung, umfassend einen pharmazeutisch annehmbaren Träger und eine pharmakologisch wirksame Menge der wie in Anspruch 1 beanspruchten Verbindung, die ausreicht, um Wehen zur Vorbereitung einer Entbindung durch Kaiserschnitt zu hemmen.

9. Die Verwendung einer wie in Anspruch 1 beanspruchten Verbindung zur Herstellung eines Medikaments zur Wehenhemmung zur Vorbereitung einer Entbindung durch Kaiserschnitt bei einem Säugetier.

10. Eine pharmazeutische Zusammensetzung, umfassend einen pharmazeutisch annehmbaren Träger und eine pharmakologisch wirksame Menge der wie in Anspruch 1 beanspruchten Verbindung, die ausreicht, um Dysmenorrhoe zu behandeln.

11. Die Verwendung einer wie in Anspruch 1 beanspruchten Verbindung zur Herstellung eines Medikaments zur Behandlung von Dysmenorrhoe bei einem Säugetier.

12. Die Verwendung einer wie in Anspruch 1 beanspruchten Verbindung zur Herstellung eines Medikaments zur Antagonisierung der Bindung von Vasopressin an seine Rezeptorstelle in einem Säugetier.

## Revendications

1. Un composé de la formule 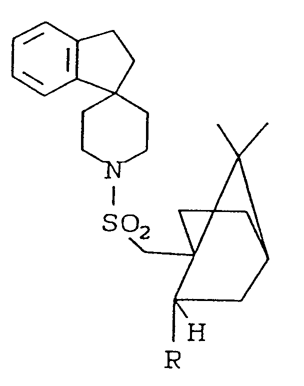 et les sels ci-dedans acceptables au plan
pharmaceutique, où,
R est
Het-R', où
Het est
noyaux hétérocycliques saturés ou non saturés substitués à 5 ou 6 membres contenant 1, 2 ou 3 hétéro-atomes, où lesdits hétéro-atomes sont M, O ou S;
R' est indépendamment un ou plusieurs d'hydrogène, oxo, thiono, amino ou alkyl non substitué ou substitué, où ledit substituant alkyl est indépendamment un ou plusieurs d'hydroxyle, amino, oxo, sulfonyle, alkyl-sulfonyle, carboxyl, phényl, indole, alcoxy-carbonyle, carbonyle-amino, guanidine, alcoxy-carbonyle-amino, imidazole, imidazolyl-alkyl-amino-carbonyle, pyrrolidine, tétrazolyl-amino-carbonyle, tétrazolyl-alkyl-carbonyle-amino, alkyl-amino-alkyl-amino-carbonyle et di-alkyl-amino-alkyl-amino-carbonyle.

2. Un composé de la formule 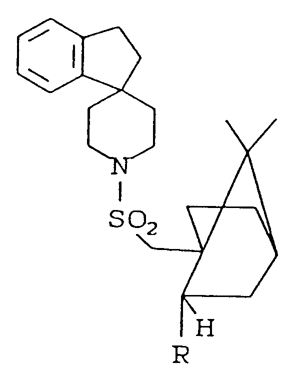 et les sels ci-dedans acceptables au plan
pharmaceutique, où,
R est
Het-R', où
Het est
noyaux hétérocycliques substitués à 5 membres contenant 1 ou 2 hétéro-atomes, où lesdits hétéro-atomes sont N;
R' est indépendamment un ou plusieurs d'hydrogène, oxo, amino ou alkyl non substitué ou substitué, où ledit substituant alkyl est indépendamment un ou plusieurs d'hydroxyle, amino, oxo, sulfonyle, alkyl-sulfonyle, carboxyle, phényl, indole, alcoxy-carbonyle, alcoxy-carbonyle-amino, imidazole ou pyrrolidine.

3. Le composé tel que déclaré dans la Prétention 1, de la formule

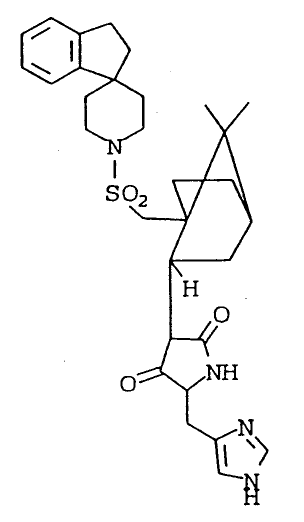

4. Une composition pharmaceutique comprenant un vecteur acceptable au plan pharmaceutique et un volume efficace au plan pharmacologique du composé, comme déclaré dans la Prétention 1, suffisant pour s'opposer la liaison de l'oxytocine et son site récepteur.

5. L'utilisation d'un composé comme déclaré dans la Prétention 1 pour la fabrication d'un médicament pour s'opposer la liaison de l'oxytocine et son site récepteur chez le mammifère.

6. Une composition pharmaceutique comportant un vecteur acceptable au plan pharmaceutique et un volume de composé efficace au plan pharmacologique, comme déclaré dans la Prétention 1, suffisant pour empêcher le travail préterminal chez le mammifère en ayant la nécessité.

7. L'utilisation d'un composé comme déclaré dans la Prétention 1, pour la fabrication d'un médicament permettant d'empêcher le travail préterminal chez le mammifère.

8. Une composition pharmaceutique comprenant un vecteur acceptable au plan pharmaceutique et un volume de composé efficace au plan pharmacologique, comme déclaré dans la Prétention 1, suffisant pour arrêter le travail pour les préparatifs de l'accouchement par césarienne.

9. L'utilisation d'un composé comme déclaré dans la Prétention 1, pour la fabrication d'un médicament permettant l'arrêt le travail pour les préparatifs de l'accouchement par césarienne chez le mammifère.

10. Une composition pharmaceutique comprenant un vecteur acceptable au plan pharmaceutique et un volume de composé efficace au plan pharmacologique, comme déclaré dans la Prétention 1, suffisant pour le traitement de la dysménorrhée.

11. L'utilisation d'un composé comme déclaré dans la Prétention 1, pour la fabrication d'un médicament pour le traitement de la dysménorrhée chez le mammifère.

12. L'utilisation d'un composé comme déclaré dans la Prétention 1 pour la fabrication d'un médicament pour s'opposer la liaison de la vasopressine et son site récepteur chez le mammifère.
